# EUROPÄISCHE PATENTANMELDUNG

(11) **EP 3 543 655 A1**
(43) Veröffentlichungstag der Anmeldung: **25.09.2019**
(21) Anmeldenummer: 19163147.2
(22) Anmeldetag: 15.03.2019
(51) Int. Cl.: G01D 4/00, G01N 25/20, G01N 33/22, F17D 1/04, G01K 17/00

(54) **VERFAHREN ZUM BESTIMMEN EINES GASVERBRAUCHS UND SYSTEM ZUR DURCHFÜHRUNG DES VERFAHRENS**

(30) Priorität: 20.03.2018 DE 102018106576
(71) Anmelder: EnBW Energie Baden-Württemberg AG, 76131 Karlsruhe (DE)
(72) Erfinder: Kessler, Alois, 75417 Mühlacker (DE)
(74) Vertreter: RPK Patentanwälte Reinhardt, Pohlmann und Kaufmann Partnerschaft mbB

(57) **Zusammenfassung**

Die Erfindung betrifft ein Verfahren zum Bestimmen eines Gasverbrauchs bei wechselnder Gasbeschaffenheit in einem Gasnetz (100), mit wenigstens einer Zählereinrichtung (20) zur Erfassung zumindest eines Gasvolumenstroms, wobei eine oder mehrere lokale Gaseinspeisungspunkte (110) im Gasnetz (100) vorgesehen sind, gekennzeichnet durch das Bestimmen zumindest von einer aktuellen Gasbeschaffenheit in einem Netzabschnitt (114) des Gasnetzes (100), wobei wenigstens eine Verbrauchsstelle (10) im Netzabschnitt (114) einen Gasvolumenstrom entnimmt; das Übermitteln zumindest der aktuellen Gasbeschaffenheit des Gases im Netzabschnitt (114) an eine Datenzentrale (60); das Bestimmen eines Verbrauchs an Gasvolumenstrom der wenigstens einen Verbrauchsstelle (10) mit deren Zählereinrichtung (20); das Übermitteln des aktuellen Verbrauchs anhand zeitgenauer Zählermesswerte der Zählereinrichtung (20) an die Datenzentrale (60); das Bestimmen des aktuellen Brennwerts anhand der Gasbeschaffenheit im Netzabschnitt (114); sowie das Verknüpfen zumindest der zeitgenauen Brennwerte mit den Zählermesswerten der Verbrauchsstelle (10).

Die Erfindung betrifft ferner ein System (200) zur Durchführung des Verfahrens.

## Beschreibung

### Stand der Technik

Die Erfindung betrifft ein Verfahren zur Bestimmung eines Gasverbrauchs insbesondere bei wechselnder Gasbeschaffenheit und ein System zur Durchführung des Verfahrens.

Die Versorgung aus überwiegend so genannten erneuerbaren Energiequellen wird zukünftig über den Stromsektor hinaus auch verstärkt die Sektoren "Wärme" und "Verkehr" betreffen. Dies ist unter dem Stichwort "Sektorkopplung" geläufig. Biogene und synthetische Gase aus erneuerbaren Energien werden hierbei eine zentrale Rolle spielen.

Die Gasabrechnung ist in der Technischen Regel DVGW G685:2008 aus dem Jahr 2008 geregelt. Für die Abrechnung der Brennwerte werden von einem Rekonstruktionssystem mittlere monatliche Brennwerte für einzelne Gasnetzabschnitte, die so genannten Brennwert-Bezirke, gebildet und an das Abrechnungssystem übermittelt. Für die kontinuierliche Überwachung der Gasbeschaffenheit werden derzeit allein in Deutschland rund 500 Messstellen eingesetzt. Eine Erweiterung des Systems, um der wachsenden Zahl dezentraler Gaseinspeisungspunkte zukünftig gewachsen zu sein, erfordert hohe Investitionen.

Bisher werden bei der Einspeisung erneuerbarer Gase quasistationäre Bedingungen eingestellt, so dass ein möglichst konstanter zeitlicher Verlauf der Gasbeschaffenheit erreicht werden kann. Durch eine Konditionierungsanlage zur Zudosierung von beispielsweise Propan wird ein stabiler Brennwert sichergestellt, auf dessen Basis später die monatliche oder jährliche Abrechnung erfolgt.

Es sind verschiedene Verfahren bekannt, um die Gasbeschaffenheit für Abrechnungszwecke zu bestimmen. Beispielsweise offenbart die EP 967483 A2 ein Berechnungsverfahren, bei dem die Schallgeschwindigkeit des Gases unter Referenzbedingungen bestimmt und der Brennwert aus einer der Messgrößen Dielektrizitätskonstante, Schallgeschwindigkeit unter weiteren Referenzbedingungen aus den erfassten Daten extrahiert wird.

### Offenbarung der Erfindung

Die Aufgabe der Erfindung ist die Schaffung eines Verfahrens, das die Bestimmung eines Gasverbrauchs bei wechselnder Gasbeschaffenheit vereinfacht.

Weiterhin soll ein System zur Durchführung des Verfahrens geschaffen werden.

Die Aufgaben werden durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Die Erfindung geht aus von einem Verfahren zum Bestimmen eines Gasverbrauchs bei wechselnder Gasbeschaffenheit in einem Gasnetz, mit wenigstens einer Zählereinrichtung zur Erfassung zumindest eines Gasvolumenstroms, wobei eine oder mehrere lokale Gaseinspeisungspunkte im Gasnetz vorgesehen sind.

Es wird vorgeschlagen, dass zumindest eine aktuelle Gasbeschaffenheit in einem Netzabschnitt des Gasnetzes bestimmt wird, wobei wenigstens eine Verbrauchsstelle im Netzabschnitt einen Gasvolumenstrom entnimmt. Zumindest die aktuelle Gasbeschaffenheit des Gases im Netzabschnitt wird an eine Datenzentrale übermittelt. Ein Verbrauch an Gasvolumenstrom der wenigstens einen Verbrauchsstelle wird mit deren Zählereinrichtung bestimmt. Der aktuelle Verbrauch wird anhand zeitgenauer Zählermesswerte der Zählereinrichtung an die Datenzentrale übermittelt. Der aktuelle Brennwert wird anhand der Gasbeschaffenheit im Netzabschnitt bestimmt. Zumindest die zeitgenauen Brennwerte werden mit den Zählermesswerten der Verbrauchsstelle verknüpft.

Vorteilhaft können zumindest die zeitgenauen Brennwerte an die Verbrauchsstelle übermittelt werden und die Verknüpfung mit den Zählermesswerten der Verbrauchsstelle dort erfolgen.

Die Gasbeschaffenheit kann die Gaszusammensetzung umfassen, die beispielsweise mit Gaschromatographen bestimmt werden kann. Daraus kann der Brennwert abgeleitet werden. Optional kann zusätzlich oder alternativ ein Kalorimeter eingesetzt werden, welches den aktuellen Brennwert des Gases bestimmen kann. Das Gasnetz führt überwiegend Erdgas (Methan), wobei an den Gaseinspeisungspunkten andere Gase wie Wasserstoff, Biogas und dergleichen dem Erdgas beigemischt werden.

Der Brennwert wird durch die chemische Zusammensetzung des Gases im Netzabschnitt bestimmt. Vorzugsweise können lokale Parameter, beispielsweise Temperatur und/oder Druck, herangezogen werden, die sich beispielsweise als brennwertrelevante Zählermesswerte aus der Zählereinrichtung, neben den Verbrauchsdaten des Gasvolumenstroms, entnehmen lassen.

Für die langfristig zu erwartende volatile und dezentrale Einspeisung erneuerbarer Gase in das Gasnetz entsprechend ihrer Verfügbarkeit, beispielsweise über erneuerbare Energien, wie Windenergie, Solarenergie etc., wird ein angepasstes Messverfahren geschaffen, das mehr Flexibilität erlaubt und gleichzeitig einen Verzicht auf eine Brennwert-Konditionierung im Netzabschnitt ermöglicht.

Dies erlaubt eine korrekte Abrechnung von Verbräuchen bei Endkunden auch bei schwankenden Gasqualitäten ohne ausgleichende Konditionierung. Ebenso kann die Einspeisung und korrekte Abrechnung unterschiedlichster Gase, wie Erdgas, Bioerdgas, Wasserstoff, ermöglicht werden. Sofern derartige Gase nicht überwiegend aus Methan bestehen, ändern sich die transporttechnischen und brenntechnischen Eigenschaften des Gasgemisches teilweise erheblich, wie beispielsweise Verbrennungsgeschwindigkeit, Dichte, Brennwert, Viskosität.

Auch die zunehmende Vermischung von niederkalorischem Gas ("L-Gas", Schwachgas) und hochkalorischem Gas ("H-Gas") durch wechselnde Bezugsquellen wird in einigen Gasnetzgebieten zu einer stärkeren Volatilität der Gasbeschaffenheit im Gasnetz beitragen. Selbst bei einer konstanten Schwachgas-Einspeisung ergibt sich durch den saisonal unterschiedlichen Gasverbrauch im Jahresverlauf eine veränderliche Zusammensetzung des sich bildenden Gasgemisches.

Durch die Vermeidung bekannter alternativer Maßnahmen, z. B. Messtechnik oder Brennwertanpassung durch Konditionierung, insbesondere durch Zumischen von Propan, sind Kosteneinsparungen beim Betreiber möglich. Kunden können bei Fragen zu relevanten Gaskennwerten, wie etwa Wobbe-Index, Methanzahl und dergleichen unterstützt werden. Ferner ist eine genaue Kenntnis der zeitlichen und örtlichen Verteilung der Gasqualität im gesamten Gasnetz möglich. Dies kann vorteilhaft für industrielle Prozesse genutzt werden, bei denen Gas aus dem Netzabschnitt eingesetzt wird.

Der Wobbe-Index ist ein Kennwert für die Austauschbarkeit von Gasen hinsichtlich der Wärmebelastung der Gasgeräte. Er wird in der Regel auf den Normzustand bezogen. Brenngase unterschiedlicher Zusammensetzung zeigen bei gleichem Wobbe-Index und unter gleichem Druck (Fließdruck) am Brenner der Verbrauchsstelle eine annähernd gleiche Wärmebelastung.

Vorzugsweise ist die Zählereinrichtung mit einem so genannten Gateway zur Datenübermittlung an die Datenzentrale ausgerüstet. Das Gateway kann in der Zählereinrichtung integriert sein oder außerhalb der Zählereinrichtung an der Verbrauchsstelle vorhanden sein. Elektronische Zähler sind günstigerweise geeignet, um ein Normvolumen des verbrauchten Gases zu erfassen. Das Gateway dient dazu, eine Kommunikation zwischen der Zählereinrichtung und der Datenzentrale zu ermöglichen.

Nach einer günstigen Ausgestaltung können jeweils zeitgenaue Verbrauchsdaten und Brennwerte an der oder durch die Verbrauchsstelle lokal bereitgestellt werden. Die Verbrauchsdaten umfassen zumindest die Zählermesswerte, die den verbrauchten Gasvolumenstrom wiedergeben. Darüber hinaus können die Verbrauchsdaten auch Parameter wie Druck des Gases und/oder Temperatur des Gases und/oder Uhrzeit des Verbrauchs, umfassen.

Insbesondere können die zeitgenauen Zählermesswerte am Ort der Verbrauchsstelle, insbesondere in der Zählereinrichtung, gespeichert werden. Vorteilhaft weist die Zählereinrichtung dazu einen geeigneten Speicherbereich auf. Die Zählereinrichtung kann optional mit einem Display ausgestattet sein, um die Zählermesswerte graphisch darzustellen. Eine Darstellung kann auch alternativ oder zusätzlich auf Displays eines Smartphones, eines Computers, eines Tabletcomputers oder dergleichen erfolgen. Vorzugsweise ist die Zählereinrichtung mit einem Zeitsignal gekoppelt, um eine zeitgenaue Zuordnung von verbrauchten Gasvolumina zu ermöglichen. Das Gateway kann hierbei beispielsweise der Zeitsynchronisation dienen, um Schaltzeiten in der Zählereinrichtung mit einer gesetzlichen Zeit (z.B. gesetzlicher Zeitstandard der Physikalisch Technischen Bundesanstalt PTB in Braunschweig) zu synchronisieren.

Die Zählereinrichtung umfasst vorzugsweise ein Kommunikationsmodul, welches zum Datenaustausch mit einem Kommunikationskanal gekoppelt ist. Das Kommunikationsmodul dient allgemein als Plattform für einen Transfer von Daten zwischen einem zählerspezifischen Datenprotokoll, z.B. Messdaten eines Industriezählers entsprechend einem Industrieprotokoll, und einem Protokoll, welches für den Kommunikationskanal geeignet ist, vorzugsweise ein IP-Protokoll (Internetprotokoll).

Vorteilhaft kann die Zählereinrichtung als Webclient konfiguriert sein. Bevorzugt kann die Zählereinrichtung einen Webclient mit einem Push-Dienst umfassen. Auf diese Weise kann die Zählereinrichtung selbsttätig mit der Datenzentrale in Kontakt treten und Zählermesswerte oder auch andere Daten übermitteln.

Zusätzlich kann die Zählereinrichtung als Webserver konfiguriert sein und insbesondere eine Webseite für die Verbrauchsstelle bereitstellen. Über diese Webseite kann der Nutzer bequem Verbrauchsdaten abfragen und verfolgen. Dies kann über einen Computer erfolgen oder auch über eine separate Anzeigeeinheit, etwa einen kleinen tragbaren Computer wie einem PDA (PDA = Personal Digital Assistant), oder auch einem Mobiltelefon. Der Webserver dient zur Übermittlung von Daten, die vorzugsweise in kurzen Abständen Verbrauchsdaten lokal für den Nutzer bereitstellen. Optional kann der Nutzer zusätzlich oder alternativ die Daten über die Datenzentrale abfragen.

Optional kann die Zählereinrichtung in ein lokales Netzwerk (LAN) in der Verbrauchsstelle eingebunden sein. So kann der Nutzer mit einem Heimcomputer auf die Zählereinrichtung zugreifen und z.B. Daten vom Kommunikationsmodul abfragen. Ebenso können über das lokale Netzwerk Verbrauchsdaten an eine lokale Anzeigeeinheit übertragen werden.

Nach einer günstigen Ausgestaltung kann eine Bereitstellung von Zählermessewerten der Verbrauchsstelle in engen Zeitabständen erfolgen, vorzugsweise wenigstens in Form von Stundenwerten, insbesondere in Form von Bruchteilen von Stundenwerten. Üblicherweise werden Gasverbräuche in wesentlich größeren Zeitabständen, etwa einmal im Jahr, erfasst und bewertet. Durch die kürzeren Zeitabstände kann eine wechselnde Beschaffenheit des Gases korrekt erfasst, bewertet und ggf. auch visualisiert werden. Die Zeitabstände können konstant sein oder bedarfsweise angepasst werden, etwa verkürzt oder verlängert werden. Vorteilhaft können die Zeitabstände abhängig von der Menge des eingespeisten Gases und/oder der Art des eingespeisten Gases und/oder der Entnahmemenge angepasst werden.

Nach einer günstigen Ausgestaltung können die Zählermesswerte von der Datenzentrale bedarfsabhängig abgerufen werden. Die Datenzentrale kann das Datenaufkommen zweckmäßig steuern und aus den abgerufenen Zählermesswerten den Verbrauch und die Kosten für den Nutzer extrahieren.

Nach einer günstigen Ausgestaltung können die aktuellen Zählermesswerte in vorgegebenen Zeitabständen an die Datenzentrale gesendet werden. Dies erfolgt vorzugsweise mit hochaufgelösten Daten, um einen lokalen Verbrauch visualisieren zu können.

Nach einer günstigen Ausgestaltung können zur hochaufgelösten Echtzeitdarstellung von aktuellen Verbrauchsdaten die aktuellen Zählermesswerte lokal bei der Verbrauchsstelle gespeichert und visualisiert werden. Insbesondere kann die Datenzentrale zur Echtzeitdarstellung aktuelle Brennwerte und/oder Kosten an die Verbrauchsstelle senden. Ein Verbraucher kann sich über seinen aktuellen Gasverbrauch und die Kosten lokal informieren. Vorteilhaft kann der Verbraucher den Gasverbrauch der Verbrauchsstelle zumindest bereichsweise kostenoptimiert steuern.

Nach einer günstigen Ausgestaltung kann das Bestimmen der aktuellen Brennwerte auf der Basis von Analysen der Gaszusammensetzung im Netzabschnitt, der Messung der Brennwerte mittels beispielsweise Kalorimetern und/oder auf der Basis von Strömungssimulationsrechnungen im Netzabschnitt erfolgen. Insbesondere können Strömungssimulationsrechnungen im Netzabschnitt abhängig vom Vorhandensein von Pendelzonen im Netzabschnitt erfolgen. Zweckmäßigerweise sind die eingespeisten Gasmengen sowie die Entnahmemengen aus dem Netzabschnitt zu berücksichtigen.

Nach einer günstigen Ausgestaltung können ein oder mehrere Verbraucher an der Verbrauchsstelle abhängig von den aktuellen Brennwerten des Gases im Netzabschnitt gesteuert oder geregelt betrieben werden. Dies ist besonders vorteilhaft bei industriellen Prozessen, bei denen mit Gas geheizt wird und bei denen die Temperatur in engen Grenzen eingehalten werden muss. Dies ist beispielsweise bei der Glasherstellung der Fall. Günstigerweise kann der Prozess anhand der aktuellen Brennwerte des Gases geführt werden.

Nach einem weiteren Aspekt der Erfindung wird ein System zur Durchführung eines erfindungsgemäßen Verfahrens vorgeschlagen, bei dem in einem Netzabschnitt eines Gasnetzes Analysemittel zum Bestimmen einer Gasbeschaffenheit vorgesehen sind. Ferner ist wenigstens eine Verbrauchsstelle im Netzabschnitt mit einer elektronischen Zählereinrichtung vorhanden. Weiterhin sind Übertragungsmittel zum Datenaustausch zwischen einer Datenzentrale und der wenigstens einen Verbrauchsstelle vorhanden.

Zur Bestimmung der Gasbeschaffenheit kann ein Chromatograph zur Bestimmung der Gaszusammensetzung und/oder ein Kalorimeter zur Bestimmung des Brennwerts eingesetzt werden. Ferner können Temperatursensoren und/oder Drucksensoren vorgesehen sein, um weitere Parameter des Gases zu erfassen. Die elektronische Zählereinrichtung lässt sich aufgrund ihres Messprinzips durch Kalibration unproblematisch zur Messung eines Normvolumenstroms verwenden, d.h. eines Gasvolumenstroms unter Normbedingungen von Druck und Temperatur, während übliche mechanische Zähler nur den Betriebsvolumenstrom erfassen können, der zur Abrechnung erst in einen Normvolumenstrom umgerechnet werden muss.

Nach einer günstigen Ausgestaltung kann der wenigstens eine Gaseinspeisungspunkt in einem Transportnetz oder in einem Verteilnetz angeordnet sein. Je nach Platzierung des oder der Gaseinspeisungspunkte können geeignete Modelle zur Bestimmung der Gasbeschaffenheit herangezogen werden. Dabei kann berücksichtigt werden, wenn abhängig von Betriebsparametern eine Mischzone zwischen Erdgas und an Gaseinspeisungspunkten eingespeistem Gas wie Schwachgas oder Wasserstoff in der Gasleitung pendelt und zum Teil sogar in Gegenrichtung strömen kann.

Typischerweise unterscheidet man in einem Gasnetz zwischen Transportnetzen mit hohem Druck, Verteilnetzen mit Mitteldruck, typischerweise mit etwa 1 bar Überdruck, und Ortsnetzen mit niedrigem Druck, typischerweise mit bis zu 0,1 bar Überdruck. Die verschiedenen Netze sind üblicherweise miteinander verknüpft. So kann beispielsweise ein Verteilnetz an ein oder an mehrere Transportnetze angeschlossen sein. Daher kann die Gasbeschaffenheit empfindlich von der Position der Gaseinspeisungspunkte abhängen. Daneben sind Einflüsse wie Einspeisemenge, Entnahmemenge und dergleichen zu beachten.

Nach einer günstigen Ausgestaltung kann wenigstens ein Elektrolyseur an einem Gaseinspeisungspunkt zur Einspeisung von Wasserstoff in den Netzabschnitt vorgesehen sein. Vorteilhaft kann mittels des Elektrolyseurs überschüssiger elektrischer Strom aus Windenergie und/oder Sonnenenergie zur Herstellung von Wasserstoff genutzt und damit indirekt gespeichert werden. Der Wasserstoff kann in das Gasnetz eingeleitet werden. Das Gasnetz selbst bildet einen Speicher für Wasserstoff und damit indirekt für elektrischen Strom. Aus Wetterdaten und Wettervorhersagedaten kann optional eine zu erwartende Wasserstoffmenge abgeleitet und insbesondere vorausschauend abgeleitet werden. Zweckmäßigerweise kann eine Bestimmung eines aktuellen Brennwerts des Gases basierend auf derartigen Prozessen getriggert werden. Insbesondere können Zeitabstände, in denen der Brennwert ermittelt wird, daran angepasst werden.

Nach einer günstigen Ausgestaltung können Mittel zum Bestimmen einer Gasbeschaffenheit, insbesondere einer Gaszusammensetzung, an einem Gaseinspeisungspunkt und/oder stromab des Gaseinspeisungspunkts vorgesehen sein. Vorteilhaft können Gaschromatographen und/oder Kalorimeter als derartige Mittel vorgesehen sein.

Nach einer günstigen Ausgestaltung kann wenigstens eine Schwachgasquelle, beispielsweise eine Biogasanlage, zur Einspeisung von Schwachgas in den Netzabschnitt vorgesehen sein. Vorteilhaft kann mittels der Schwachgasquelle überschüssiger elektrischer Strom aus Windenergie und/oder Sonnenenergie zur Herstellung von Schwachgas genutzt und damit indirekt im Gasnetz gespeichert werden. Das Gasnetz selbst bildet einen Speicher für das Schwachgas und damit indirekt für elektrischen Strom. Aus Wetterdaten und Wettervorhersagedaten kann optional eine zu erwartende Wasserstoffmenge abgeleitet und insbesondere vorausschauend abgeleitet werden. Zweckmäßigerweise kann eine Bestimmung eines aktuellen Brennwerts des Gases basierend auf derartigen Prozessen getriggert werden. Insbesondere können Zeitabstände, in denen der Brennwert ermittelt wird, daran angepasst werden.

Nach einer günstigen Ausgestaltung kann wenigstens ein Rechenmittel zur Simulation einer Gasbeschaffenheit, insbesondere Gaszusammensetzung und/oder Brennwert, vorgesehen sein. Diese kann in der Datenzentrale angeordnet sein oder bei der Messanalytik zur Bestimmung der Gaszusammensetzung oder einer externen Einheit und mit der Datenzentrale kommunizieren.

### Zeichnung

Weitere Vorteile ergeben sich aus der folgenden Zeichnungsbeschreibung. In den Zeichnungen sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### Es zeigen beispielhaft:

- Fig. 1: ein Blockdiagramm mit einem Ausführungsbeispiel der Erfindung, mit einem Gaseinspeisungspunkt und einer in einem Netzabschnitt stromab angeordneten Verbrauchsstelle mit einer Zählereinrichtung;
- Fig. 2: eine schematische Darstellung eines Gasnetzes mit Transportnetzen, Verteilnetzen und Ortsnetzen;
- Fig. 3: schematisch ein Blockdiagramm mit einem Gaseinspeisungspunkt in ein Transportnetz nach einem Ausführungsbeispiel der Erfindung;
- Fig. 4: schematisch ein Blockdiagramm mit einem Gaseinspeisungspunkt in einem Verteilnetz nach einem weiteren Ausführungsbeispiel der Erfindung;
- Fig. 5: schematisch ein Blockdiagramm mit einem Gaseinspeisungspunkt nach einem Ausführungsbeispiel der Erfindung mit einer Einspeisung stromab einer Verbrauchsstelle mit Mischzone;
- Fig. 6: schematisch ein Blockdiagramm mit zweiseitiger Einspeisung aus zwei Transportnetzen in ein Verteilnetz und mit einem Gaseinspeisungspunkt in einem der Transportnetze nach einem Ausführungsbeispiel der Erfindung;
- Fig. 7: schematisch ein Blockdiagramm mit zweiseitiger Einspeisung aus zwei Transportnetzen in ein Verteilnetz und mit einem Gaseinspeisungspunkt in ein Verteilnetz stromauf einer Verbrauchsstelle nach einem Ausführungsbeispiel der Erfindung;
- Fig. 8: schematisch ein Blockdiagramm mit zweiseitiger Einspeisung aus zwei Transportnetzen in ein Verteilnetz und mit einem Gaseinspeisungspunkt in ein Verteilnetz stromab einer Verbrauchsstelle nach einem Ausführungsbeispiel der Erfindung.

### Ausführungsformen der Erfindung

In den Figuren sind gleichartige oder gleichwirkende Komponenten mit gleichen Bezugszeichen beziffert. Die Figuren zeigen lediglich Beispiele und sind nicht beschränkend zu verstehen.

Im Folgenden verwendete Richtungsterminologie mit Begriffen wie "links", "rechts", "oben", "unten", "davor" "dahinter", "danach" und dergleichen dient lediglich dem besseren Verständnis der Figuren und soll in keinem Fall eine Beschränkung der Allgemeinheit darstellen. Die dargestellten Komponenten und Elemente, deren Auslegung und Verwendung können im Sinne der Überlegungen eines Fachmanns variieren und an die jeweiligen Anwendungen angepasst werden.

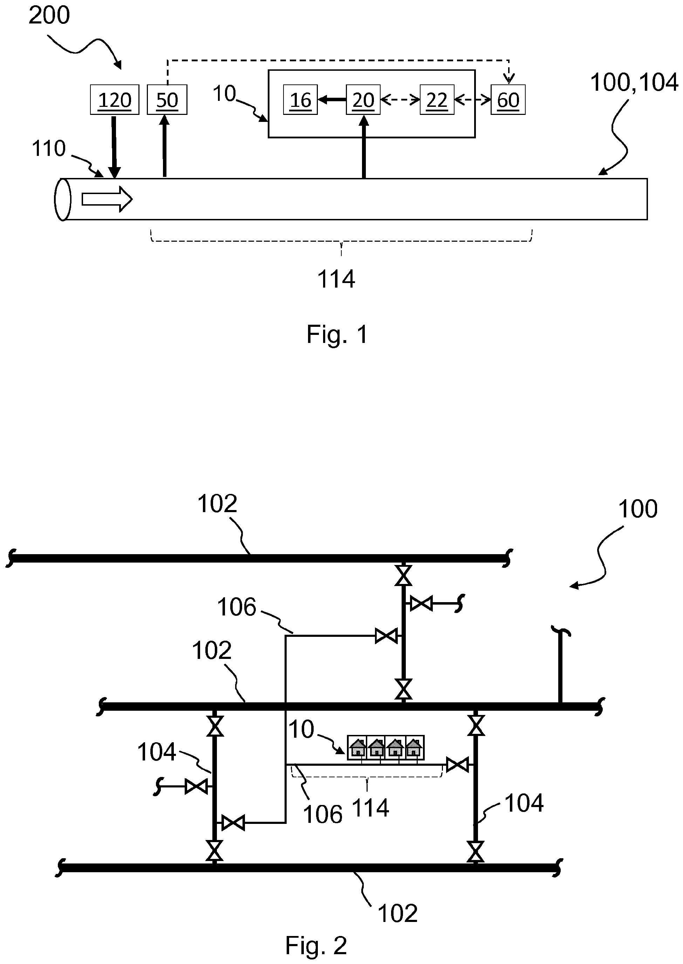

Figur 1 zeigt ein Blockdiagramm mit einem Ausführungsbeispiel der Erfindung, mit einem Verteilnetz 104 eines Gasnetzes 100, in dem Erdgas zu Verbrauchsstellen 10 transportiert wird. Figur 2 zeigt eine schematische Darstellung des Gasnetzes 100 mit Transportnetzen 102, Verteilnetzen 104 und Ortsnetzen 106.

An einem Gaseinspeisungspunkt 110 (Figur 1) wird Wasserstoff in ein Verteilnetz 104 eines Gasnetzes 100 eingespeist, der durch einen Elektrolyseur 120 aus Wasser erzeigt wird. Vorzugsweise wird zum Betrieb des Elektrolyseurs 120 elektrischer Strom aus Wind- oder Sonnenenergie eingesetzt. Die erzeugte Menge an Wasserstoff ist dann nicht konstant, sondern entspricht der verfügbaren, insbesondere überschüssigen, Strommenge, die nicht vom normalen Stromversorgungsnetz aufgenommen werden kann.

Der erzeugte Wasserstoff kann vorteilhaft im Gasnetz 100 gespeichert werden. Statt Wasserstoff aus einem Elektrolyseur 120 kann auch anderes Gas eingespeist werden, insbesondere Schwachgas aus einer Biogasanlage oder Klärgasanlage, aus Deponien oder mittels thermischen Prozessen aus Biomasse erzeugtes Gas. Während Wasserstoff einen hohen Brennwert aufweist, hat Schwachgas einen deutlich geringeren Brennwert wie Methan. Ein Blockpfeil symbolisiert die Gasströmung im Verteilnetz 104.

Eine Verbrauchsstelle 10 ist stromab des Gaseinspeisungspunkts 110 in einem Netzabschnitt 114 des Verteilnetzes 104 angeordnet. Die Verbrauchsstelle 10 kann über ein nicht dargestelltes Ortsnetz an das Verteilnetz 104 angeschlossen sein. Der als Zusatzgas eingespeiste Wasserstoff muss, im Gegensatz zu Schwachgasen, nicht aufbereitet oder konditioniert werden. Bis zum Ausspeisepunkt an der Verbrauchsstelle 10 ist der Wasserstoff mit dem Erdgas in der Gasleitung (Verteilnetz 104) hinreichend vermischt.

Der Elektrolyseur 120 speist Wasserstoff volatil, d.h. mit schwankenden Mengen, in die Gasleitung (Verteilnetz 104) ein. Eine Analyseeinrichtung 50, insbesondere ein Gaschromatograph, misst periodisch die Gaszusammensetzung am Gaseinspeisungspunkt 110 und übermittelt diese Werte mit Zeitstempel an eine Datenzentrale 60.

An der Verbrauchsstelle 10, beispielsweise einem Wohngebäude, bezieht ein Endgerät 16 einen Volumenstrom an Gas mit volatiler Gasbeschaffenheit aus der Gasleitung (Verteilnetz 104). Der Volumenstrom an Gas passiert zuerst die Zählereinrichtung 20 und gelangt dann zum Endgerät 16. Die Gasströmung aus dem Verteilnetz 104 zum Endgerät 16 ist mit einem dicken Pfeil symbolisiert.

Das Endgerät 16 kann ein Kessel sein, ein Gasherd oder dergleichen. Optional kann das Endgerät 16 auch eine industrielle Einrichtung sein, wie etwa eine gewerbliche Gas-Tankstelle oder ein sonstiger gewerblicher Gasverbraucher.

Die Zählereinrichtung 20 der Verbrauchsstelle 10 ermittelt den aktuellen Gasvolumenstrom zum Endgerät 16. Die elektronische Zählereinrichtung 20 ist so kalibriert, dass deren Zählermesswerte den Normvolumenstrom des verbrauchten Gases unter Normbedingungen von Druck und Temperatur wiedergeben und mit einem Zeitstempel versehen werden, so dass eine zeitgenaue Messung des Gasverbrauchs erfolgt. Eine Verbindungskomponente in Form eines Gateways 22 übermittelt die Zählermesswerte entsprechend des gemessenen Volumenstroms an die Datenzentrale 60. Diese berechnet aus dem Volumenstrom und der Gasbeschaffenheit den Brennwert und den Geldwert des Gasbezugs im Abrechnungszeitintervall. Neben den verbrauchsbezogenen Zählermesswerten können auch brennwertrelevante Zählermesswerte wie etwa Druck und/oder Temperatur und/oder lokale Wetterprognosen und dergleichen an die Datenzentrale 60 übermittelt werden.

Die Datenzentrale 60 übermittelt zeitgenaue Brennwerte an die Verbrauchsstelle 10. Die zeitgenauen Brennwerte können beispielsweise aus den Werten generiert werden, welche die Analyseeinrichtung 50, insbesondere ein Gaschromatograph, beispielsweise durch periodische Messung der Gaszusammensetzung am Gaseinspeisungspunkt 110 gemessen und mit Zeitstempel an die Datenzentrale 60 übermittelt hat.

An der Verbrauchsstelle 10 kann die Zählereinrichtung 20 jeweils zeitgenaue Verbrauchsdaten und Brennwerte der Verbrauchsstelle 10 darstellen.

Die Zählermesswerte können von der Datenzentrale 60 in festen oder flexiblen Zeitabständen, beispielsweise stündlich oder in kürzeren Abständen, bedarfsweise abgerufen werden (pull-Betrieb). Zusätzlich oder alternativ können die aktuellen Zählermesswerte von der Zählereinrichtung 20 über das Gateway 22 in vorgegebenen Zeitabständen an die Datenzentrale 60 gesendet werden.

Mit Vorteil können die aktuellen Zählermesswerte zur hochaufgelösten Echtzeitdarstellung von aktuellen Verbrauchsdaten lokal bei der Verbrauchsstelle 10 gespeichert und visualisiert werden. Zur Echtzeitdarstellung kann die Datenzentrale 60 aktuelle Brennwerte und/oder Kosten an die Verbrauchsstelle 10 senden. Dies kann beispielsweise dann erfolgen, wenn Zählermesswerte an die Datenzentrale 60 übermittelt werden.

Das Bestimmen der aktuellen Brennwerte kann auf der Basis von Analysen der Gaszusammensetzung im Netzabschnitt 114 und/oder auf der Basis von Strömungssimulationsrechnungen im Netzabschnitt 114 erfolgen.

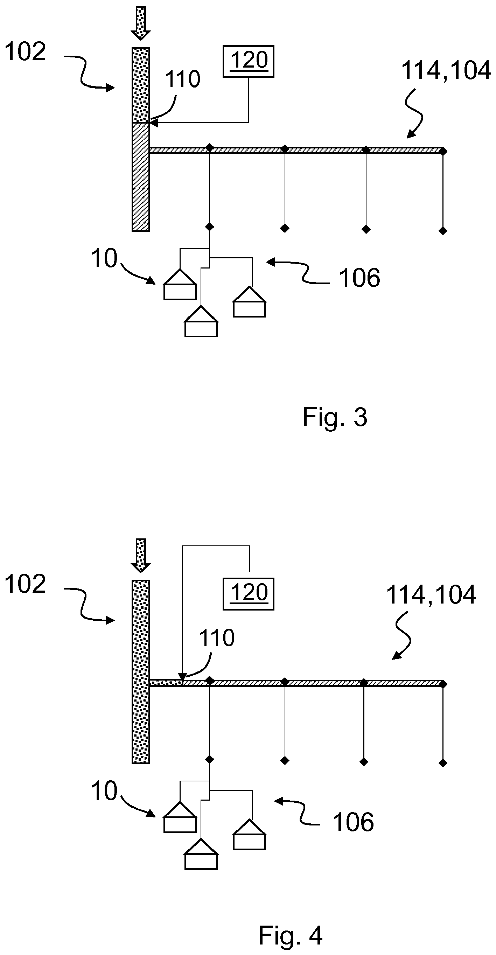

Die Figuren 3 und 4 zeigen schematisch Situationen, bei denen die Verhältnisse hinsichtlich der Gasbeschaffenheit bei der Verbrauchsstelle 10 klar definiert sind. Dabei ist eine einseitige Einspeisung aus einem Transportnetz 102 in ein Verteilnetz 104 oder Ortsnetz 106 gegeben und ein Gaseinspeisungspunkt 110 liegt stromauf der Verbrauchsstelle 10.

Figur 3 zeigt dabei ein Blockdiagramm mit einem Gaseinspeisungspunkt 110 in einem Transportnetz 102, während in Figur 4 an einem Gaseinspeisungspunkt 110 Gas in ein Verteilnetz 104 eingespeist wird. Das Verteilnetz 104 ist nur mit einem Transportnetz 102 verbunden, so dass eine Gasströmungsrichtung aufgrund der Druckverhältnisse in Transportnetz 102 und Verteilnetz 104 eindeutig und Richtung Verbrauchsstelle 10 gerichtet ist.

Elektronische Zählereinrichtungen 20 sind bei entsprechender Kalibrierung und aufgrund des angewandten Messprinzips in der Lage, den Normvolumenstrom zu messen. Damit besteht die Möglichkeit, dass die zur Visualisierung benötigte Umrechnung mit dem Brennwert und die Berechnung der Gasbezugskosten anhand des aktuellen Gaspreises lokal an der Verbrauchsstelle 10 erfolgen kann. Brennwert und Gaspreis werden sodann von der zentralen Datenzentrale 60 übermittelt. Hochaufgelöste Verbrauchskurven können daher aus den lokalen Zählermesswerten gebildet werden, während nur die abrechnungsrelevanten Stunden- oder Viertelstunden-Zählermesswerte an die zentrale Datenzentrale 60 zu übermitteln sind. Mit Vorteil sendet die Datenzentrale 60 zu diesen Übermittlungszeitpunkten die aktuellen Brennwerte an die Verbrauchsstelle 10.

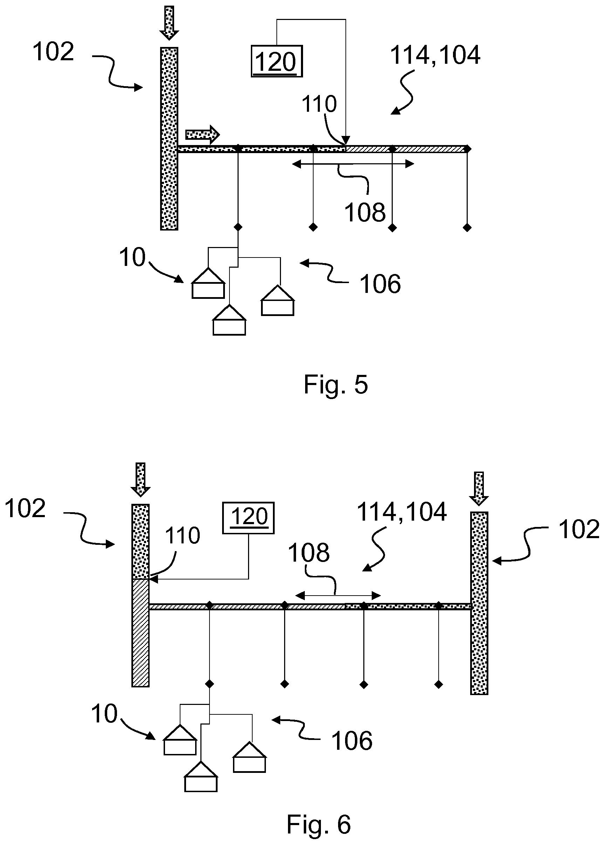

In den Figuren 5 bis 8 sind Verhältnisse skizziert, bei denen ein Pendeln von Mischzonen 108 im Gasnetz 100 möglich ist, was zu zeitlich stark fluktuierenden unterschiedlichen lokalen Gaszusammensetzungen an den Verbrauchsstellen 10 führen kann.

Figur 5 zeigt schematisch ein Blockdiagramm eines Gasnetzes 100 mit einem Gaseinspeisungspunkt 110 eines Elektrolyseurs 120 nach einem Ausführungsbeispiel der Erfindung mit einer Einspeisung von Wasserstoff in ein Verteilnetz 104 stromab einer Verbrauchsstelle 10. Dabei bildet sich eine Mischzone 108 aus, in der Erdgas aus dem einseitig an das Verteilnetz 104 angekoppelten Transportnetz 102 mit dem an dem Gaseinspeisungspunkt 110 eingespeisten Wasserstoff gemischt wird. Die Mischzone 108 kann im Verteilnetz 104 hin und her wandern, je nach Einspeisemenge in das Verteilnetz 104, Entnahmemenge an der Verbrauchsstelle 10, Druckverhältnissen und Strömungsverhältnissen im Transportnetz 102 und dergleichen. Die Gaszusammensetzung an der Verbrauchsstelle 10 kann zeitlich und räumlich stark fluktuieren.

Figur 6 zeigt schematisch ein Blockdiagramm eines Gasnetzes 100 mit einem Gaseinspeisungspunkt 110 eines Elektrolyseurs 120 nach einem Ausführungsbeispiel der Erfindung mit einer Einspeisung von Wasserstoff in ein Transportnetz 102 stromauf einer Verbrauchsstelle 10 in einem Verteilnetz 104. Das Verteilnetz 104 ist jeweils an beiden Seiten an ein Transportnetz 102 angeschlossen. Dabei bildet sich eine Mischzone 108 aus, in der Erdgas aus dem in der Figur linken Transportnetz 102 mit dem an dem Gaseinspeisungspunkt 110 eingespeisten Wasserstoff gemischt wird. Die Mischzone 108 kann im Verteilnetz 104 hin und her wandern, je nach Einspeisemenge am Gaseinspeisungspunkt 110, Entnahmemenge an der Verbrauchsstelle 10, Druckverhältnissen und Strömungsverhältnissen in den beiden Transportnetzen 102 und dergleichen. Die Gaszusammensetzung kann an der Verbrauchsstelle 10 zeitlich und räumlich stark fluktuieren.

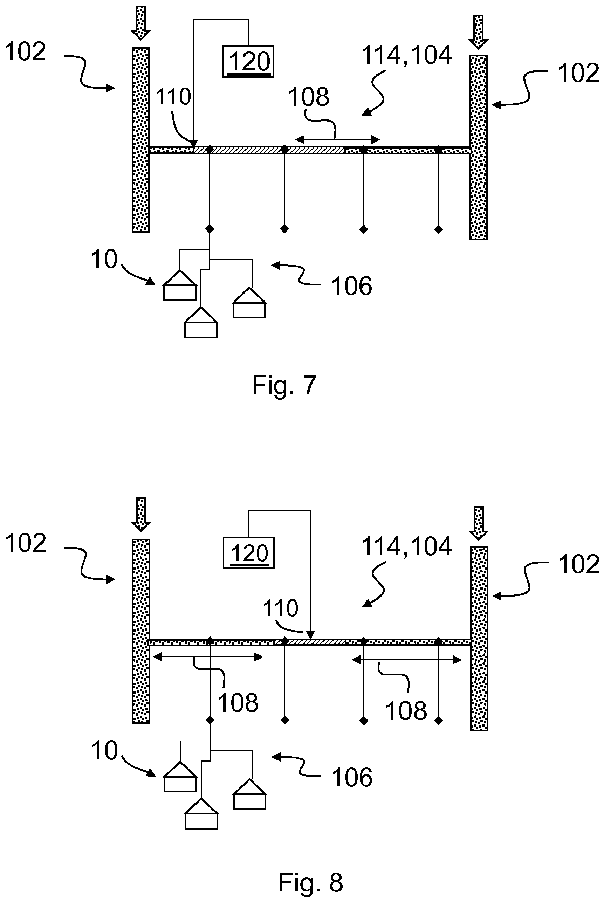

Figur 7 zeigt schematisch ein Blockdiagramm eines Gasnetzes 100 mit einem Gaseinspeisungspunkt 110 eines Elektrolyseurs 120 nach einem Ausführungsbeispiel der Erfindung mit einer Einspeisung von Wasserstoff in ein Verteilnetz 104 stromauf von Verbrauchsstellen 10, die in einem Ortsnetz 106 angeordnet sind. Der Gaseinspeisungspunkt 110 liegt dabei zwischen dem in der Figur links dargestellten Transportnetz 102 und der Verbrauchsstelle 10. Das Verteilnetz 104 ist jeweils an beiden Seiten an ein Transportnetz 102 angeschlossen. Dabei bildet sich eine Mischzone 108 im Verteilnetz 104 aus, in der Erdgas aus beiden Transportnetzen 102 mit dem an dem Gaseinspeisungspunkt 110 eingespeisten Wasserstoff gemischt wird. Die Verbrauchsstelle 10 erhält eine Mischung von Wasserstoff aus dem Elektrolyseur 120 und Erdgas aus einem oder beiden der Transportnetze 102. Die Mischzone 108 kann im Verteilnetz 104 hin und her wandern, je nach Einspeisemenge am Gaseinspeisungspunkt 110, Entnahmemenge an der Verbrauchsstelle 10, Druckverhältnissen und Strömungsverhältnissen in den beiden Transportnetzen 102 und dergleichen. Die Gaszusammensetzung kann an der Verbrauchsstelle 10 zeitlich und räumlich stark fluktuieren.

Figur 8 zeigt schematisch ein Blockdiagramm eines Gasnetzes 100 mit einem Gaseinspeisungspunkt 110 eines Elektrolyseurs 120 nach einem Ausführungsbeispiel der Erfindung mit einer Einspeisung von Wasserstoff in ein Verteilnetz 104 stromab einer Verbrauchsstelle 10 in dem Verteilnetz 104. Der Gaseinspeisungspunkt 110 liegt stromab von an ein Ortsnetz 106 angeschlossenen Verbrauchsstellen 10 zwischen den beiden Transportnetzen 102. Das Verteilnetz 104 ist jeweils an beiden Seiten an ein Transportnetz 102 angeschlossen. Dabei bilden sich zwei Mischzonen 108 im Verteilnetz 104 aus, in der Erdgas aus beiden Transportnetzen 102 mit dem an dem Gaseinspeisungspunkt 110 eingespeisten Wasserstoff gemischt wird. Die Mischzonen 108 können im Verteilnetz 104 hin und her wandern, je nach Einspeisemenge am Gaseinspeisungspunkt 110, Entnahmemenge an der Verbrauchsstelle 10, Druckverhältnissen und Strömungsverhältnissen in den beiden Transportnetzen 102 und dergleichen. Die Gaszusammensetzung kann an der Verbrauchsstelle 10 zeitlich und räumlich stark fluktuieren.

Je nach Position des Gaseinspeisungspunkts 110 pendelt die Mischzone in den Ausführungsbeispielen der Figuren 5 bis 8, was zu volatilen Brennwerten und entsprechenden Abrechnungsproblemen führen kann.

Insbesondere bei Vorliegen von pendelnden Mischzonen 108 im Netzabschnitt 114, optional auch ohne solche pendelnden Mischzonen 108, werden in größeren Netzabschnitten 114 vorzugsweise Simulationstools eingesetzt, um anhand von Simulationen zeitgenaue Brennwerte zu bestimmen. Dies kann mit oder ohne hin und her pendelnde Mischzonen 108 erfolgen. Vorteilhaft werden Strömungssimulationsrechnungen im Netzabschnitt 114 abhängig vom Vorhandensein und/oder Änderungen von pendelnden Mischzonen 108 im Netzabschnitt 114 durchgeführt. Hierzu sind entsprechende Rechenmittel zur Simulation einer Gaszusammensetzung oder eines Brennwerts vorgesehen.

Vorteilhaft kann vorgesehen sein, dass ein oder mehrere Gas verbrauchende Endgeräte an der Verbrauchsstelle 10 abhängig von den aktuellen Brennwerten des Gases im Netzabschnitt 114 gesteuert oder geregelt betrieben werden.

So können industrielle Prozesse, beispielsweise bei der Glasherstellung, insbesondere von Flachglas, mit höherer Sicherheit ausgeführt werden. Das Glas wird mit einer Gasflamme erhitzt und muss in einem engen Temperaturfenster gehalten werden. Ändert sich der Brennwert des Gases während des Prozesses, ist dies für das Produkt abträglich. Ist dagegen bekannt, dass sich der Brennwert ändert bzw. ändern wird, kann der Brenner an den geänderten Brennwert des Gases angepasst werden. Günstigerweise kann eine Änderung des Brennwerts mitgeteilt werden, bevor das Gas mit geändertem Brennwert an der Verbrauchsstelle 10 ankommt. Dies kann mit einem ausreichenden Vorsprung erfolgen, der für eine Einstellung des Brenners ausreichend ist.

## Patentansprüche

1. Verfahren zum Bestimmen eines Gasverbrauchs bei wechselnder Gasbeschaffenheit in einem Gasnetz (100), mit wenigstens einer Zählereinrichtung (20) zur Erfassung zumindest eines Gasvolumenstroms, wobei eine oder mehrere lokale Gaseinspeisungspunkte (110) im Gasnetz (100) vorgesehen sind,
**gekennzeichnet durch**
(i) Bestimmen zumindest von einer aktuellen Gasbeschaffenheit in einem Netzabschnitt (114) des Gasnetzes (100), wobei wenigstens eine Verbrauchsstelle (10) im Netzabschnitt (114) einen Gasvolumenstrom entnimmt;
(ii) Übermitteln zumindest der aktuellen Gasbeschaffenheit des Gases im Netzabschnitt (114) an eine Datenzentrale (60);
(iii) Bestimmen eines Verbrauchs an Gasvolumenstrom der wenigstens einen Verbrauchsstelle (10) mit deren Zählereinrichtung (20);
(iv) Übermitteln des aktuellen Verbrauchs anhand zeitgenauer Zählermesswerte der Zählereinrichtung (20) an die Datenzentrale (60);
(v) Bestimmen des aktuellen Brennwerts anhand der Gasbeschaffenheit im Netzabschnitt (114);
(vi) Verknüpfen zumindest der zeitgenauen Brennwerte mit den Zählermesswerten der Verbrauchsstelle (10).

2. Verfahren nach Anspruch 1, **dadurch gekennzeichnet, dass** jeweils zeitgenaue Verbrauchsdaten und Brennwerte an der oder durch die Verbrauchsstelle (10) lokal bereitgestellt werden.

3. Verfahren nach Anspruch 1 oder 2, **dadurch gekennzeichnet, dass** die zeitgenauen Zählermesswerte in der Verbrauchsstelle (10) und/oder der Zählereinrichtung (20) gespeichert werden.

4. Verfahren nach einem der vorhergehenden Ansprüche, **dadurch gekennzeichnet, dass** eine Bereitstellung von Zählermesswerten der Verbrauchsstelle (10) in engen Zeitabständen erfolgt, vorzugsweise wenigstens in Form von Stundenwerten, insbesondere in Form von Bruchteilen von Stundenwerten.

5. Verfahren nach einem der vorhergehenden Ansprüche, **dadurch gekennzeichnet, dass** die Zählermesswerte von der Datenzentrale (60) bedarfsabhängig abgerufen werden.

6. Verfahren nach einem der vorhergehenden Ansprüche, **dadurch gekennzeichnet, dass** die aktuellen Zählermesswerte in vorgegebenen Zeitabständen an die Datenzentrale (60) gesendet werden.

7. Verfahren nach einem der vorhergehenden Ansprüche, **dadurch gekennzeichnet, dass** zur hochaufgelösten Echtzeitdarstellung von aktuellen Verbrauchsdaten die aktuellen Zählermesswerte lokal bei der Verbrauchsstelle (10) gespeichert und visualisiert werden.

8. Verfahren nach einem der vorhergehenden Ansprüche, **dadurch gekennzeichnet, dass** zur hochaufgelösten Echtzeitdarstellung von aktuellen Verbrauchsdaten die Datenzentrale (60) aktuelle Brennwerte und/oder Kosten an die Verbrauchsstelle (10) sendet.

9. Verfahren nach einem der vorhergehenden Ansprüche, **dadurch gekennzeichnet, dass** das Bestimmen der aktuellen Brennwerte auf der Basis von Analysen der Gaszusammensetzung im Netzabschnitt (114) und/oder auf der Basis von Strömungssimulationsrechnungen im Netzabschnitt (114) erfolgt.

10. Verfahren nach Anspruch 9, **dadurch gekennzeichnet, dass** Strömungssimulationsrechnungen im Netzabschnitt (114) abhängig vom Vorhandensein von Pendelzonen im Netzabschnitt (114) erfolgen.

11. Verfahren nach einem der vorhergehenden Ansprüche, **dadurch gekennzeichnet, dass** ein oder mehrere Verbraucher an der Verbrauchsstelle (10) abhängig von den aktuellen Brennwerten des Gases im Netzabschnitt (114) gesteuert oder geregelt betrieben werden.

12. System (200) zur Durchführung eines Verfahrens nach einem der vorhergehenden Ansprüche,
**dadurch gekennzeichnet, dass**
(i) in einem Netzabschnitt (114) eines Gasnetzes (100) Mittel zum Bestimmen einer Gasbeschaffenheit vorgesehen sind;
(ii) wenigstens eine Verbrauchsstelle (10) im Netzabschnitt (114) mit einer elektronischen Zählereinrichtung (20) vorhanden ist;
(iii) Übertragungsmittel (22) zum Datenaustausch zwischen einer Datenzentrale (60) und der wenigstens einen Verbrauchsstelle (10) vorhanden sind.

13. System nach Anspruch 12, **dadurch gekennzeichnet, dass** wenigstens ein Gaseinspeisungspunkt (110) in einem Transportnetz (102) oder in einem Verteilnetz (104) angeordnet ist.

14. System nach Anspruch 12 oder 13, **dadurch gekennzeichnet, dass** wenigstens ein Elektrolyseur (120) an einem Gaseinspeisungspunkt (110) zur Einspeisung von Wasserstoff in den Netzabschnitt (114) vorgesehen ist.

15. System nach einem der Ansprüche 12 bis 14, **dadurch gekennzeichnet, dass** Mittel (50) zum Bestimmen einer Gasbeschaffenheit, insbesondere einer Gaszusammensetzung, an einem Gaseinspeisungspunkt (110) und/oder stromab des Gaseinspeisungspunkts (110) vorgesehen sind.

16. System nach einem der Ansprüche 12 bis 15, **dadurch gekennzeichnet, dass** wenigstens eine Schwachgasquelle, insbesondere eine Biogasanlage, zur Einspeisung von Schwachgas in den Netzabschnitt (114) vorgesehen ist.

17. System nach einem der Ansprüche 12 bis 16, **dadurch gekennzeichnet, dass** wenigstens ein Rechenmittel zur Simulation einer Gasbeschaffenheit, insbesondere Gaszusammensetzung, vorgesehen ist.
